# EUROPEAN PATENT SPECIFICATION

(11) **EP 0 922 060 B1**
(45) Date of publication and mention of the grant of the patent: **30.10.2002**
(21) Application number: 97940137.9
(22) Date of filing: 28.08.1997
(51) Int. Cl.: C08B 37/08, A61L 17/00

(54) **HYALURONIC ACID ESTERS, THREADS AND BIOMATERIALS CONTAINING THEM, AND THEIR USE IN SURGERY**
ESTER VON HYALURONSÄURE, SIE ENTHALTENDE FADEN UND BIOMATERIALEN, IHRE VERWENDUNG IN DER CHIRURGIE
ESTERS DE L'ACIDE HYALURONIQUE, FILS ET BIOMATERIAUX LES CONTENANT, ET LEUR UTILISATION EN CHIRURGIE

(30) Priority: 29.08.1996 IT PD960207
(43) Date of publication of application: 16.06.1999
(73) Proprietor: FIDIA ADVANCED BIOPOLYMERS, S.R.L., 35031 Abano Terme (Padova) (IT)
(72) Inventor: CALLEGARO, Lanfranco, I-36016 Thiene (IT); BELLINI, Davide, I-35036 Montegrotto Terme (IT)
(74) Representative: Gervasi, Gemma, Dr.
(86) International application number: EP9704684
(87) International publication number: WO98008876

(56) References cited:
- EP-A- 0 216 453
- WO-A-95/24429
- WO-A-97/07833
- PATENT ABSTRACTS OF JAPAN vol. 18, no. 236 (C-1196), 6 May 1994 & JP 06 025306 A (SHISEIDO CO LTD), 1 February 1994,

## Description

### TECHNICAL FIELD

The present invention concerns the preparation of a new series of ester derivatives of hyaluronic acid, biodegradable threads essentially constituted by such derivatives, and their use in the fields of medicine and surgery.

### BACKGROUND ART

Suture threads are now widely used in modern surgical practice and can be made of a wide range of materials, according to the type of surgery to be performed (Abraham R. Katz et al. "A new synthetic monofilament absorbable suture made from polytrimethylene carbonate" Surgery, Gynecology & Obstetrics, September 1985, vol. 161, pages 213-222; Abraham R. Katz et al. "Evaluation of tensile and absorption properties of polyglycolic acid sutures" Surgery, Gynecology & Obstetrics, October 1970, vol. 131, pages 701-716). It is possible, therefore, to imagine different types of suture thread with different characteristics of gauge, tensile strength, biocompatibility and biodegradability, according to whether they are intended for extensive lacerations (abdominal wall, thorax, lower limbs), or for small cuts and wounds as on the face, mouth and soft tissues. Some conditions require the material to be biocompatible but not biodegradable (as in cardiovascular surgery), while others necessitate both these characteristics (as in surgery to the urinary tract). The suture threads currently on the market vary first and foremost in the type of polymer with which they are made. Indeed, they vary from non-reabsorbable threads based on polyester, polypropylene, nylon and silk, such as Surgilene® , Surgilon® , Novafil® and Dermalon® by DG (Davis + Geck - American Cyanamid Company), to reabsorbable threads based on glycolic acid and collagen, such as Vicryl® and Catgut® by Ethicon (A. Pavan et al. "A Comparative Study of Poly Glycolic acid and Catgut as Suture Materials. Histomorphology and Mechanical Properties", Journal of Biomedical Materials Research, vol. 13, pages 477-496, 1979). As these materials all have a synthetic polymeric matrix, they are poorly biocompatible and only some of them are biodegradable, so they may cause inflammatory reactions at the lesion site where they are applied (E. A. Bakkum et al. "Quantitative analysis of the inflammatory reaction surrounding sutures commonly used in operative procedures and the relation to postsurgical adhesion formation" Biomaterials 1995, vol. 16, No. 17, pages 1283-1289) and may necessitate a second surgical operation to remove them from the application site.

The PCT Application No. WO95/24429 discloses derivatives of carboxy polysaccharides, comprising hyaluronic acid, wherein all or part of the carboxy groups thereof are esterified with an aromatic alcohol, a substituted aromatic alcohol, an aromatic heterocyclic alcohol, a substituted aromatic heterocyclic alcohol, a N-hydroxylamine or a combination thereof, and discloses also their use in the biomedical and pharmaceutical fields to prepare, for example, cosmetic articles, healthcare articles, surgical articles, and diagnostic kits.

The European Patent Application No. 216 453 discloses esters of hyaluronic acid, wherein all or part of the carboxy groups thereof, are esterified with alcohols of the aliphatic, araliphatic, cycloaliphatic or heteroaliphatic series and salts of the partial esters; pharmaceutical preparations containing, as an active ingredient, one or more among these hyaluronic acid esters or a salt thereof; and medicaments containing a pharmacologically active substance or an association of pharmacologically active substances and a carrier containing a total or partial ester of hyaluronic acid.

Lastly, the use of ester derivatives of hyaluronic acid is known in the preparation of biomaterials, including suture threads, in the medical-surgical sector (European Patents EP 341745 and EP 216453)

### BRIEF DESCRIPTION OF THE FIGURES

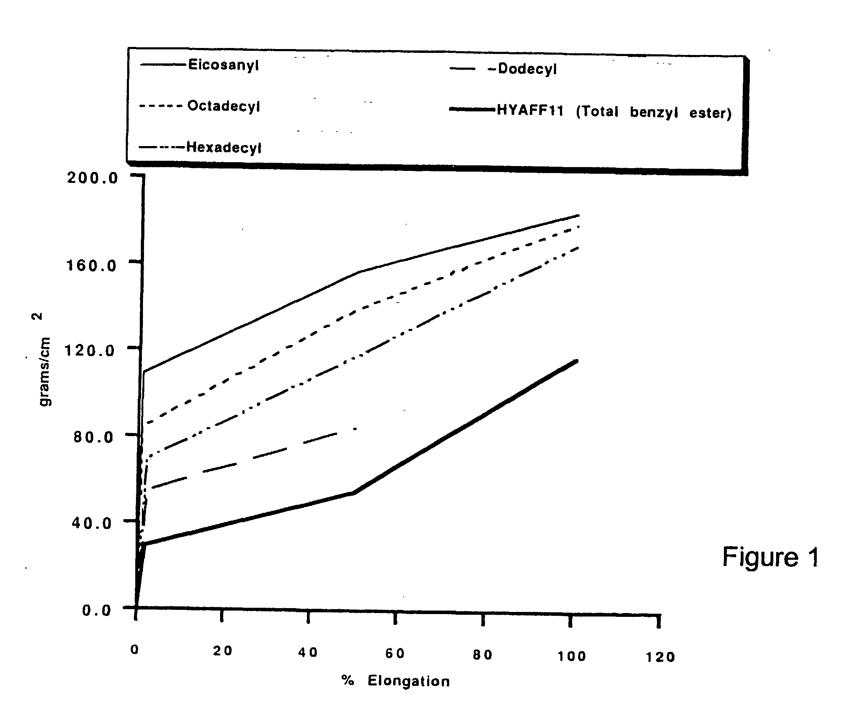

Figure 1: Testing the tensile properites of hyaluronic acid esters according to the present invention.

"Eicosanyl": hyaluronic acid derivative with 75% of its carboxy functions esterified with benzyl alcohol, 20% esterified with eicosanyl alcohol (arachidyl alcohol; CH₃(CH₂)₁₈-CH₂-OH) and the remaining 5% salified with sodium.

"Octadecyl": hyaluronic acid derivative with 75% of its carboxy functions esterified with benzyl alcohol and the remaining 25% esterified with octadecyl alcohol (stearyl alcohol; CH₃-(CH₂)₁₆-CH₂-OH).

"Hexadecyl": hyaluronic acid derivative with 75% of its carboxy functions esterified with benzyl alcohol (and the remaining 25% esterified with hexadecyl alcohol (cetyl palmityl alcohol; CH₃-(CH₂)₁₄-CH₂-OH).

"Dodecyl": hyaluronic acid derivative with 75% of its carboxy functions esterified with benzyl alcohol (C₆H₅-CH₂OH) and the remaining 25% esterified with dodecyl alcohol (Lauril alcohol; CH₃-(CH₂)₁₀-CH₂-OH).

HYAFF11: total ester of hyaluronic acid with benzylic alcohol (reference compound).

Figure 2: testing the tensile properites of hyaluronic acid esters according to the present invention.

Figure 3: Testing the dry tensile resistance of the multifilament made with a hyaluronic acid ester derivative with 75% of its carboxy functions esterified with benzyl alcohol and the remaining 25% esterified with octadecyl alcohol (HY11+octadecyl), compared with that of the multifilament based on the totally esterified benzyl ester (Hyaff 11).

Figure 4: Testing the wet tensile resistance of the threads made with a hyaluronic acid ester derivative with 75% of its carboxy functions esterified with benzyl alcohol and the remaining 25% esterified with dodecyl alcohol (HY11+dodecyl), and of the threads made with a hyaluronic acid ester derivative with 75% of its carboxy functions esterified with benzyl alcohol and the remaining 25% esterified with octadecyl alcohol (HY11+octadecyl), compared with that of the threads based on totally esterified benzyl and ethyl esters (Hyaff 11 and Hyaff 7, respectively).

Figure 5: comparing the tensile resistance of hyaluronic acid derivatives. "HYAFF 11": multifilament thread made of the total benzylic ester of hyaluronic acid.

"EICOSANOL": multifilament thread made of the ester of hyaluronic acid with 75% of its carboxy functions esterified with benzyl alcohol, 20% esterified with eicosanyl alcohol and the remaining 5% salified with sodium.

"CATGUT" chromic collagen monofilament for surgical suture.

Figure 6: resistance to tension one week after implant.

Figure 7: resistance to tension two weeks after implant.

### DISCLOSURE OF THE INVENTION

The present invention describes new ester derivatives of hyaluronic acid, wherein the first part of the carboxy functions is esterified with an araliphatic alcohol, such as benzyl alcohol, and the second part with long-chain, straight aliphatic alcohols with between 10 and 22 carbon atoms.

The hyaluronic acid which can be used in the present invention may be derived from any source, for example it may be obtained by extraction from rooster combs (EP 0138572; WO 92/18543), by fermentation (WO95/04132) or by biotechnological means (WO 95/24497), and its molecular weight can range between 10,000 and 10,000,000 Da, particularly between 150,000 and 1,000,000 Da.

The long-chain aliphatic alcohols are those with a straight chain between 10 and 22 carbon atoms. The increase in the number of carbon atoms in the alkyl chain and the number of carboxy functions involved in the esterification with the abovesaid alcohols, yields ester derivatives of hyaluronic acid with an increasingly high degree of lipophilia generally leading to hydrophobic interactions when they come into contact with solutions or biological fluids, with the result that the tensile strength varies from one product to another as does the biodegradability time, according to the length of the lipid alcohol introduced.

Moreover the combination of the aliphatic and araliphatic esters on the hyaluronic acid molecule allows to obtain compounds showing good biodegradability and at the same time a significant medium-term tensile strength.

The extent of esterification with aliphatic alcohols may vary from 1 to 50%, and in particular between 10 and 25%. The extent of esterification with araliphatic alcohol may vary from 50 and 75%. A preferred araliphatic alcohol is benzylic alcohol.

The esterification with aliphatic and araliphatic alcohols may involve the totality or part of the available carboxy functions of hyaluronic acid. In the latter case, the remaining non-esterified carboxy functions are salified with alkaline, alkaline earth metals and quaternary ammonium salts. Sodium in particular is used.

The long alkyl chains introduced, with between 10 and 22 carbon atoms, give the ester derivatives of hyaluronic acid tensile properties never observed before and not foreseeable in other hyaluronic acid-based thread forms.

Indeed, besides having a biocompatible and biodegradable polysaccharide matrix, thus belonging to that class of compounds which, like hyaluronic acid, have bioplastic and pharmaceutical properties, they can be given varying degrees of lipophilia according to the use they are intended for. Their lipophilia can be adjusted by modulating the insertion of a lipid chain starting from the ester matrix itself (benzyl ester of hyaluronic acid, 50 to 75% esterified)

Indeed, the increase in the lipid chain of the polymer (from C10 to C22) gives the material a structure with greater hydrophobic characteristics and modulates its degradation over time.

The present invention also comprises biodegradable threads essentially costituted by the esters described above.

The preparation of these threads first involves the synthesis of such esters. This can be done by esterification of a first part of the carboxy functions of hyaluronic acid with araliphatic alcohol, esterification of a second part of the carboxy functions of hyaluronic acid with C10-C22 straight akyl chain alcohols, and salification of the possible remaining carboxy functions not involved in the esterification steps.

The remaining steps to form the esters into threads are those commonly available in the field of thread preparation, e.g. via extrusion techniques. An application of these techniques is shown in the experimental part, example 1.

The threads can be used as suture, or for the preparation of gauzes, non-woven fabrics, meshes, tubes, and products composed of associations of such forms, which present different degrees of biodegradability and residence times, as needed. Given the excellent biodegradability of threads made of these esters, it is possible to avoid operating a second time to remove them, as is normally the case with the suture threads on the market.

Especially when present in the form of surgical suture threads, the threads are characterized by a multifilament conformation. and a tensile strength which varies, according to the ester derivative used, between 300 and 1800 g/cm². The threads preferably have a diameter which varies between 75 and 800 micron.

The main characteristic of these materials is their tensile strength which can be obtained on the basis of the following parameters:
- the molecular weight of the starting hyaluronic acid;
- the type of long-chain aliphatic alcohol used in the second esterification step;
- the percentage of carboxy groups involved in the esterification reaction with the long-chain lipid alcohol.

Figure 1 shows the different tensile properties of an ester derivative with benzyl alcohol of hyaluronic acid (Hyaff 11) from those of the derivatives of the present invention in a wet environment (saline solution), particularly as the substituted alkyl chain increases (dodecyl alcohol; hexadecyl alcohol; octadecyl alcohol; eicosanyl alcohol).

The threads thus constituted can be used to advantage in surgery, such as in maxillofacial surgery, in suture to tissues requiring a long degradation time, as in the case of materials which come into constant contact with biological fluids, or tissues requiring rapid degradation, as in the case of contact with soft tissues such as occurs in plastic surgery, as fillers in aesthetic surgery, and in dentistry.

Moreover, due to their content in hyaluronic acid derivatives, the threads according to the invention are able to act as bacteriostats and to limit the proliferation of inflammatory cells.

### EXPERIMENTAL PART

The tensile properties of the ester derivatives of hyaluronic acid have been assessed using a computerized tensiometer T-10 from MONSANTO, an instrument which can control the tensile stress applied to a given material. Generally speaking, the tensile properties of a material are measured according to its resistance to stress. When calculating tensile resistance, three main correlated values must be considered: load at break, elongation at break and shear modulus. load at break gives the amount of stress necessary to cause the thread to break.
- elongation at break is the extent to which the thread is stretched when it breaks.
- the shear modulus represents the amount of stress which must be applied before the thread begins to stretch.

The shear modulus is, therefore, correlated-with the elongation of the thread. Indeed, the greater the elastic properties of the thread, the higher the percentage of elongation at breaking point.

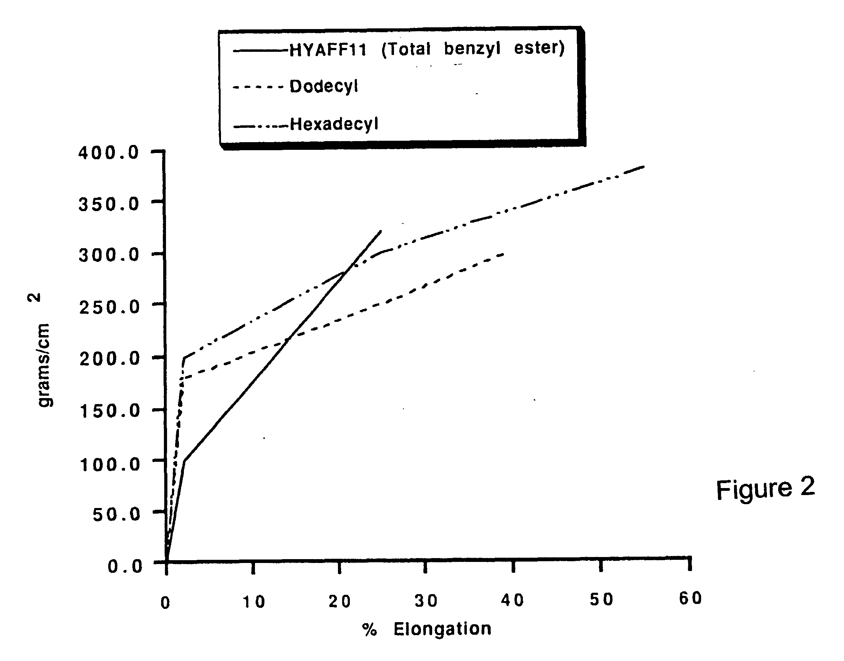

In particular, according to the variations in the lipid chain which was introduced, the ester derivatives of hyaluronic acid reported hereafter showed more marked elongation as the number of carbon atoms in the alcohol increased. Indeed, processing of the data reported in Figure 2 showed that the various hyaluronic acid ester threads presented various degrees of elongation. In the case of the benzyl ester derivative, elongation was virtually nil, while the dodecyl and hexadecyl derivatives showed an increase in elongation of the material which was proportional to the lipid chain introduced (hexadecyl>dodecyl).

### Example 1:

### Preparation of a multifilament from a hyaluronic acid derivative with 75% of its carboxy functions esterified with benzyl alcohol and the remaining 25% esterified with octadecyl alcohol (stearyl alcohol; CH₃-(CH₂)₁₆-CH₂-OH)-

The ester derivative is solubilized in DMSO to a concentration of 150 mg/ml at a temperature of 30°C. The solubilized derivative is filtered through a 20 micron mesh and placed in an extrusion reactor connected to a spinneret with 100-80 micron holes. The product is extruded in a coagulation bath containing a solvent which allows the DMSO to be extracted from the product (for example, ethanol), and the material coming out of the spinneret is wound onto a series of drafting bobbins and blown dry.

### Example 2

### Testing the dry tensile resistance of the multifilament made with a hyaluronic acid ester derivative with 75 % of its carboxy functions esterified with benzyl alcohol and the remaining 25% esterified with octadecyl alcohol, compared with that of the multifilament based on the totally esterified benzyl ester (Hyaff 11)

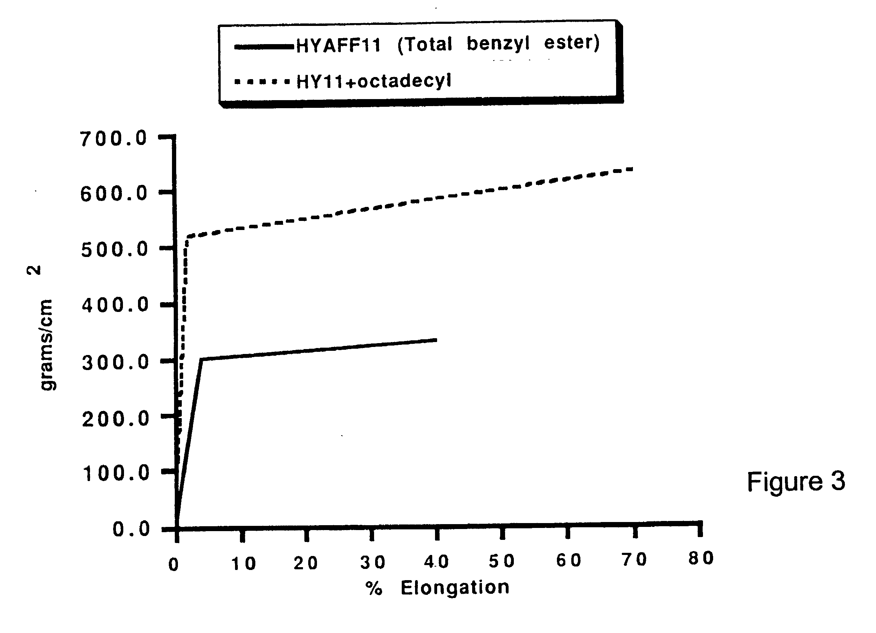

The ester derivative is processed according to the procedure described in Example 1 and the multifilament thus obtained is placed under stress to measure its tensile resistance. A T10 Tensiometer from Monsanto is used for this purpose. The results obtained are shown in Figure 3. As can be seen, the "lipid" derivative presented better resistance to stress than the multifilament based on the totally esterified benzyl ester did.

### Example 3

### Testing the wet tensile resistance of the threads made with a hyaluronic acid ester derivative with 75% of its carboxy functions esterified with benzyl alcohol and the remaining 25% esterified with octadecyl alcohol, and the threads made of a hyaluronic acid ester derivative with 75% of its carboxy functions esterified with benzyl alcohol and the remaining 25% esterified with dodecyl alcohol (lauryl alcohol; CH₃-(CH₂)₁₀-CH₂-OH), compared with that of the threads based on totally esterified benzyl and ethyl esters (Hyaff 11 and Hyaff 7, respectively).

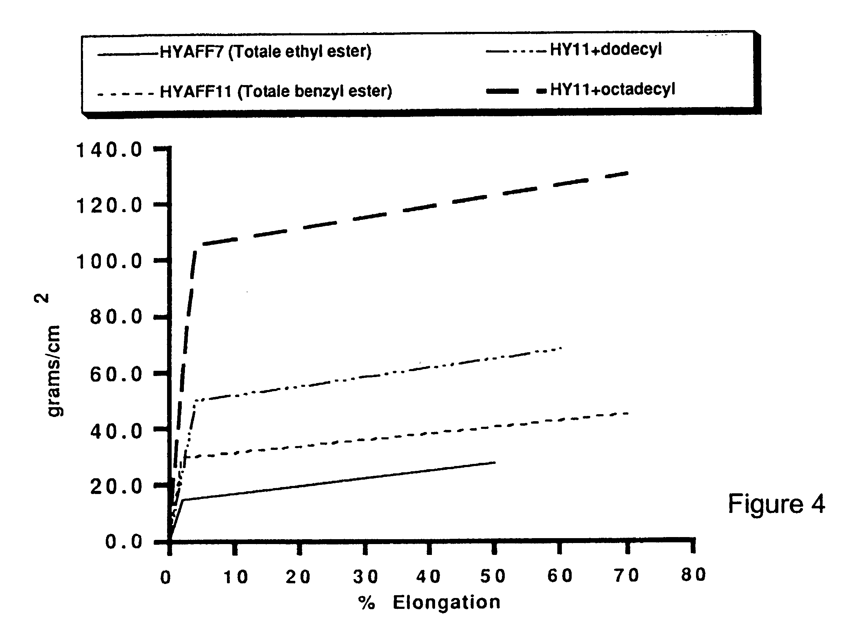

The ester derivatives are processed according to the procedure described in Example 1. The threads thus obtained are immersed for 15 hours in an aqueous solution of 0.9% NaCl w/v and then placed under stress to measure their tensile resistance. A T10 Tensiometer from Monsanto is used for this purpose. The results obtained are shown in Figure 4. As can be seen, the "lipid" derivative presented different resistance to stress, as the chain introduced was varied (dodecyl<octadecyl), from that shown by the threads obtained with the Hyaff 11 and Hyaff 7 derivatives.

### Example 4

### Testing the tensile resistance of the threads constituted by a hyaluronic acid derivative with 75% of its carboxy functions esterified with benzyl alcohol, 20% esterified with eicosanyl alcohol (arachidyl alcohol CH₃-(CH₂)₁₈-CH₂OH) and the remaining 5% salified with sodium following in vivo implantation in an animal model

### Materials:

- multifilament thread of total benzyl ester of hyaluronic acid (HYAFF 11);
- multifilament thread of the hyaluronic acid derivative with 75% of its carboxy functions esterified with benzyl alcohol, 20% esterified with eicosanyl alcohol and the remaining 5% salified with sodium (HYAFF11/p75+eicosanyl alcohol);
- chromic monofilament for surgical suture, CATGUT® (collagen);
- biocompatible and biodegradable lubricant SQUALANO, Aldrich;
- T-10 Tensiometer by Monsanto.

### Description:

Subcutaneous implant was performed on 14 S. D. Harlan rats using the following types of suture on each rat: HYAFF 11, HYAFF 11 lubricated with Squalane, HYAFF 11/p75+eicosanyl alcohol, HYAFF 11/p75 + eicosanyl alcohol lubricated with Squalane and CATGUT® commercial sutures.

The threads were lubricated with a lipophilic substance such as Squalane, a saturated aliphatic hydrocarbide of natural origin with 30 carbon atoms, to assess whether this type of treatment affords better protection from biological liquids.

The rats were subdivided into two groups and sacrificed after 7 and 14 days respectively to assess the tensile characteristics of the threads.

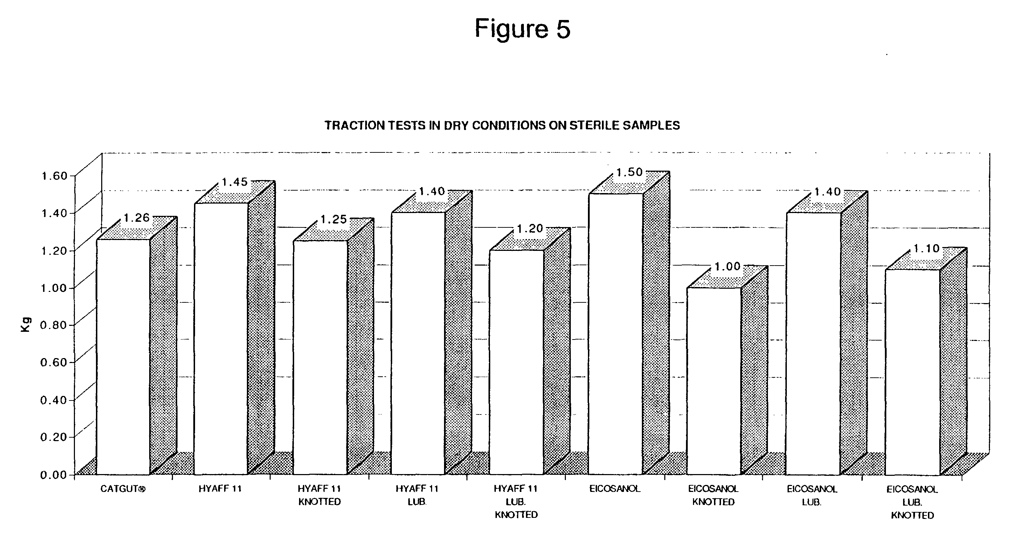

Figure 5 compares the tensile resistance of derivatives HYAFF 11 and HYAFF11/p75 + eicosanyl alcohol, both lubricated and not lubricated, with that of CATGUT_ commercial suture before implant.

The tensile characteristics of the materials are similar.

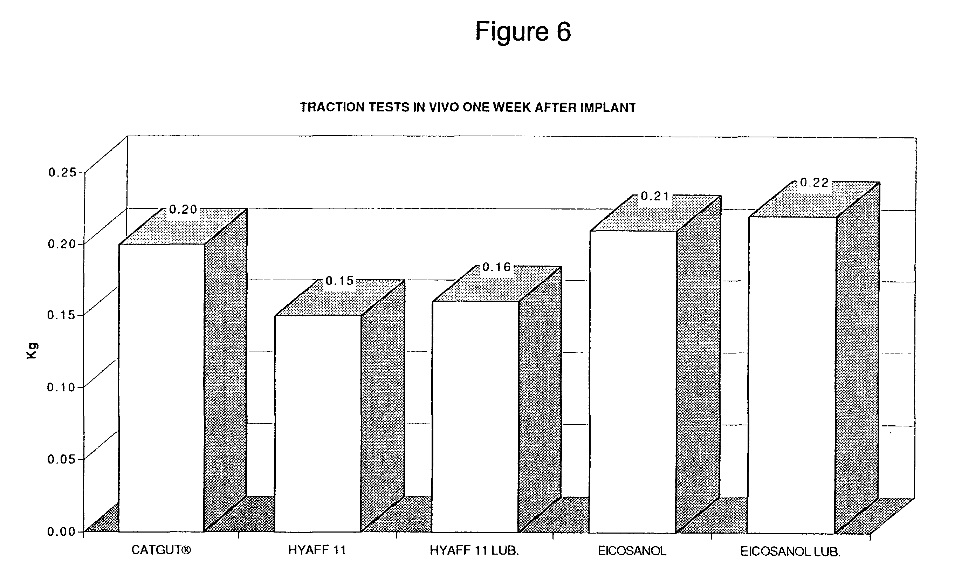

Figure 6 shows the decreased resistance to tension one week after implant. The commercial suture and that of HYAFF11/p75 + eicosanyl alcohol presented similar behaviour and the lubricated threads were the most resistant.

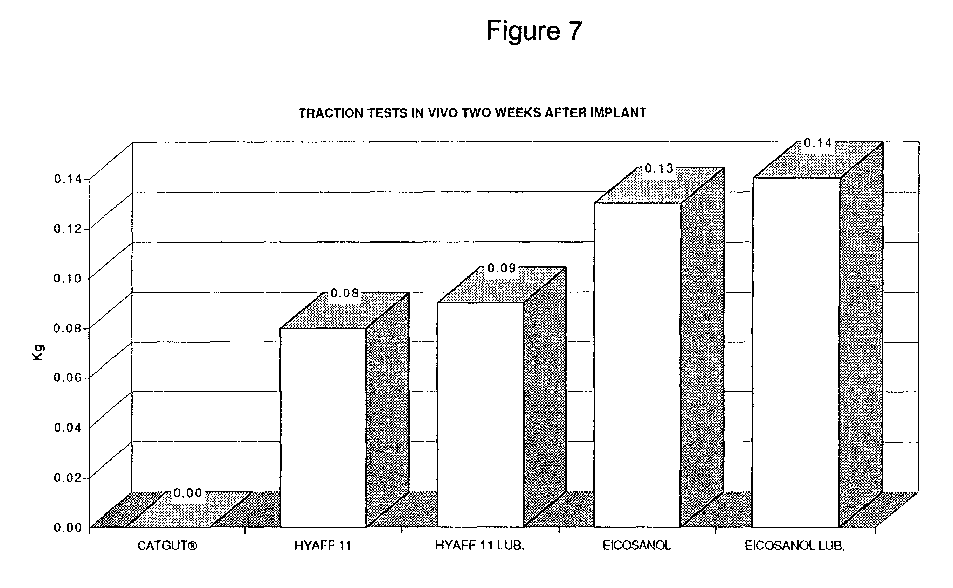

Figure 7 shows the results two weeks after implant. As can be seen, it was impossible to remove the CATGUT_ suture from the site in order to test it for tensile resistance because it was completely degraded. The threads of HYAFF11/p75 + eicosanyl alcohol, on the other hand, presented tensile resistance which was 60% greater than that of the HYAFF 11 threads.

## Claims

1. Ester derivatives of hyaluronic acid, wherein a first part of the carboxylic functions is esterified with an araliphatic alcohol, a second part is esterified with long chain, straight aliphatic alcohols with between 10 and 22 carbon atoms, and wherein the possible non esterified carboxylic functions if present are salified provided that:
when the araliphatic alcohol is benzyl alcohol, and the aliphatic alcohol is a long-chain straight aliphatic alcohols with between C₁₀-C₂₀ carbon atoms, the carboxylic functions esterified with benzyl alcohol are lower than 75%.

2. Ester derivatives according to claim 1, wherein the ester is benzyl alcohol.

3. Ester derivatives according to anyone of claims 1-2 wherein the long-chain straight alcohol is selected from the group consisting of decyl, dodecyl, hexadecyl, octadecyl, eicosyl, docosyl alcohol.

4. Ester derivatives according to anyone of claims 1-3, wherein the percentage of the carboxylic functions of hyaluronic acid esterified with araliphatic alcohols varies between 50 and 75%.

5. Ester derivatives according to anyone of claims 1-4, wherein the percentage of carboxylic functions esterified with long-chain aliphatic alcohols is comprised between 10 and 25%.

6. Ester derivatives according to anyone of claims 1-6 wherein the remaining carboxylic functions are salified with alkaline, alkaline earth metals, and quaternary ammonium salts.

7. Ester derivatives according to claim 6, wherein the remaining carboxylic functions are salified with sodium.

8. Ester derivatives according to anyone of claims 1-7, wherein the starting hyaluronic acid has a molecular weight of between 10,000 and 10,000,000 Da.

9. Ester derivatives according to claim 8 wherein hyaluronic acid has a molecular weight of between 150,000 and 1,000,000 Da.

10. Biodegradable threads having a multifilament conformation and a tensile strength comprised between 300 and 1800 g/cm² consisting essentially of ester derivatives of hyaluronic acid, wherein a first part of the carboxylic function is esterified with an araliphatic alcohol, a second part is esterified with long- chain, straight aliphatic alcohols with between 10 and 22 carbon atoms, and wherein the possible non esterified carboxylic functions, if present are salified.

11. Biodegradable threads according to claim 10, wherein the araliphatic alcohol is benzyl alcohol .

12. Biodegradable threads according to anyone of claims 10-11, wherein the long-chain straight aliphatic alcohol is chosen from the group consisting of decyl, dodecyl, hexadecyl, octadecyl, eicosyl, docosyl, alcohol.

13. Biodegradable threads according to anyone of claims 10-12, wherein the percentage of the carboxylic functions of hyaluronic acid esterified with araliphatic alcohols varies between 50 and 75%.

14. Biodegradable threads according to anyone of claims 10-13,wherein the percentage of carboxylic functions esterified with long-chain aliphatic alcohols is comprised between 10 and 25%.

15. Biodegradable threads according to anyone of claims 10-14, wherein the remaining carboxy functions are salified with alkaline, alkaline earth metals, and quaternary ammonium salts.

16. Biodegradable threads according to claim 15 wherein the remaining carboxylic functions are salified with sodium.

17. Biodegradable threads according to anyone of claims 10-16, wherein the hyaluronic acid has a molecular weight of between 10,000 and 10,000,000 Da.

18. Biodegradable threads according to anyone of claims 10-16 , wherein hyaluronic acid has a molecular weight of between 150,000 and 1,000,000 Da.

19. Biodegradable threads according to claim 10-18, having a diameter which varies between 75 and 800 microns.

20. Biodegradable threads according to claim 19, in the form of surgical suture threads.

21. Biomaterials, health-care products, and surgical articles in the form of gauzes, meshes, non-woven fabrics, tubes and association thereof containing the threads according to anyone of claims 10-19.

22. A process for preparing the biodegradable threads according to anyone of claims 10-19, comprising the following steps:
a) esterifying a first part of the carboxylic functions of hyaluronic acid with an araliphatic alcohol,
b) esterifying the possible remaining carboxylic functions with aliphatic long chain straight alcohols with between 10 and 22 carbon atoms;
c) salifying the possible remaining carboxylic functions of hyaluronic acid not involved in the preceding esterification steps,
d) subjecting the hyaluronic mixed esters obtained in step c) to conventional thread -forming processes.

23. Use of ester derivatives of hyaluronic acid, wherein a first part of the carboxylic function is esterified with an araliphatic alcohol, a second part is esterified with long-chain, straight aliphatic alcohols with between 10 and 22 carbon atoms, and wherein the possible non esterified carboxylic functions, if present are salified, for the manufacture of threads for use in general surgery, maxillofacial surgery, plastic surgery, aesthetic surgery and dentistry.

24. Use of ester derivatives of hyaluronic acid, wherein a first part of the carboxylic function is esterified with an araliphatic alcohol, a second part is esterified with long-chain, straight aliphatic alcohols with between 10 and 20 carbon atoms, and wherein the possible non esterified carboxylic functions, if present are salified, for the manufacture of threads for use as fillers in aesthetic surgery.

## Patentansprüche

1. Ester-Derivate von Hyaluronsäure, wobei ein erster Teil der carboxylischen Funktionen mit einem araliphatischen Alkohol, ein zweiter Teil mit langkettigen gerade aliphatischen Alkoholen mit 10 bis 22 Kohlenstoff-Atomen verestert wird, und wobei die möglichen nicht-veresterten carboxylischen Funktionen, wenn vorhanden, Salze bilden, vorausgesetzt, dass:
wenn der araliphatische Alkohol Benzyl-Alkohol ist und der aliphatische Alkohol ein langkettiger gerader aliphatischer Alkohol mit C₁₀ - C₂₀ Kohlenstoff-Atomen, die mit Benzyl-Alkohol veresterten Funktionen weniger als 75% betragen.

2. Ester-Derivate gemäß Anspruch 1, wobei der Ester Benzyl-Alkohol ist.

3. Ester-Derivate gemäß den Ansprüchen 1-2, wobei der langkettige gerade Alkohol aus der Gruppe bestehend aus Decyl-, Dodecyl-, Hexadecyl-, Oktadecyl-,Eicosyl-, Docosyl-Alkohol ausgewählt wird.

4. Ester-Derivate gemäß den Ansprüchen 1-3, wobei der Anteil der carboxylischen Funktionen von mit araliphatischen Alkoholen veresterter Hyaluronsäure von 50 bis 75 % variiert.

5. Ester-Derivate gemäß den Ansprüchen 1-4, wobei der Anteil an mit langkettigen aliphatischen Alkoholen veresterten carboxylischen Funktionen zwischen 10 und 25% beträgt.

6. Ester-Derivate gemäß den Ansprüchen 1-6, wobei die verbleibenden carboxylischen Funktionen mit Alkali, Erdalkalimetallen sowie quartären Ammoniumsalzen Salze bilden.

7. Ester-Derivate gemäß Anspruch 6, wobei die verbleibenden carboxylischen Funktionen mit Sodium Salze bilden.

8. Ester-Derivate gemäß den Ansprüchen 1-7, wobei die Ausgangs-Hyaluronsäure ein Molekulargewicht von 10.000 bis 10.000.000 Da aufweist.

9. Ester-Derivate gemäß Anspruch 8, wobei die Hyaluronsäure ein Molekulargewicht von 150.000 bis 1.000.000 Da aufweist.

10. Biologisch abbaubare Fäden, die eine multifile Gestalt und eine Zugfestigkeit von 300 bis 800 g/cm² aufweisen, bestehend im Wesentlichen aus Ester-Derivaten von Hyaluronsäure, wobei ein erster Teil der carboxylischen Funktionen mit einem araliphatischen Alkohol, ein zweiter Teil mit langkettigen gerade aliphatischen Alkoholen mit 10 bis 22 Kohlenstoff-Atomen verestert wird, und wobei die möglichen nicht-veresterten carboxylischen Funktionen, wenn vorhanden, Salze bilden.

11. Biologisch abbaubare Fäden gemäß Anspruch 10, wobei der araliphatische Alkohol Benzyl-Alkohol ist.

12. Biologisch abbaubare Fäden gemäß den Ansprüchen 10-11, wobei der langkettige gerade aliphatische Alkohol aus der Gruppe bestehend aus Decyl-, Dodecyl-, Hexadecyl-, Oktadecyl-, Eicosyl-, Docosyl-Alkohol ausgewählt wird.

13. Biologisch abbaubare Fäden gemäß den Ansprüchen 10-12, wobei der Anteil der mit araliphatischen Alkoholen veresterten carboxylischen Funktionen von Hyaluronsäure von 50 bis 75 % variiert.

14. Biologisch abbaubare Fäden gemäß den Ansprüchen 10-13, wobei der Anteil an mit langkettigen aliphatischen Alkoholen veresterten carboxylischen Funktionen zwischen 10 und 25% beträgt.

15. Biologisch abbaubare Fäden gemäß den Ansprüchen 10-14, wobei die verbleibenden carboxylischen Funtionen mit Alkali, Erdalkalimetallen sowie quartären Ammoniumsalzen Salze bilden.

16. Biologisch abbaubare Fäden gemäß Anspruch 15, wobei die verbleibenden carboxylischen Funtionen mit Sodium Salze bilden.

17. Biologisch abbaubare Fäden gemäß den Ansprüchen 10-16, wobei die Hyaluronsäure ein Molekulargewicht von 10.000 bis 10.000.000 Da aufweist.

18. Biologisch abbaubare Fäden gemäß den Ansprüchen 10-16, wobei die Hyaluronsäure ein Molekulargewicht von 150.000 bis 1.000.000 Da aufweist.

19. Biologisch abbaubare Fäden gemäß Ansprüchen 10-18, die einen Durchmesser von 75 bis 800 Mikrometer aufweisen.

20. Biologisch abbaubare Fäden gemäß Anspruch 19 in der Form chirurgischer Näh-Fäden.

21. Biomaterialien, Gesundheitspflege-Produkte und chirurgische Artikel in Form von Gazen, Netzen, nicht-gewebten Stoffen, Schläuchen sowie Verbindungen davon, die Fäden gemäß den Ansprüchen 10-19 enthalten.

22. Ein Verfahren zur Herstellung der biologisch abbaubaren Fäden gemäß den Ansprüchen 10-19, welches folgende Schritte umfasst:
a) Veresterung eines ersten Teils der carboxylischen Funktionen von Hyaluronsäure mit araliphatischem Alkohol,
b) Veresterung der möglichen verbleibenden carboxylischen Funktionen mit aliphatischen langkettigen geraden Alkohohlen mit 10 bis 22 Kohlenstoff-Atomen;
c) Salzbildung der verbleibenden, in die vorangegangenen Veresterungs-Schritte nicht einbezogenen carboxylischen Funktionen von Hyaluronsäure.
d) Zuführen der in Schritt c) erzielten Hyaluron-Misch-Ester konventionellen Fäden bildenden Verfahren.

23. Verwendung von Ester-Derivaten von Hyaluronsäure, wobei ein erster Teil der carboxylischen Funktionen mit einem araliphatischen Alkohol, ein zweiter Teil mit langkettigen gerade aliphatischen Alkoholen mit 10 bis 22 Kohlenstoff-Atomen verestert wird, und wobei die möglichen nicht-veresterten carboxylischen Funktionen, wenn vorhanden, Salze bilden, für die Herstellung von Fäden zum Gebrauch in allgemeiner Chirurgie, Kieferchirurgie, plastischer Chirurgie, ästhetischer Chirurgie und Zahnheilkunde.

24. Verwendung von Ester-Derivaten von Hyaluronsäure, wobei ein erster Teil der carboxylischen Funktionen mit einem araliphatischen Alkohol, ein zweiter Teil mit langkettigen gerade aliphatischen Alkoholen mit 10 bis 22 Kohlenstoff-Atomen verestert wird, und wobei die möglichen nicht-veresterten carboxylischen Funktionen, wenn vorhanden, Salze bilden, für die Herstellung von Fäden zum Gebrauch als Füller in der ästhetischen Chirurgie.

## Revendications

1. Dérivés d'ester d'acide hyaluronique, **caractérisés par le fait qu'**une première partie des fonctions carboxyles est estérifiée avec un alcool araliphatique, qu'une deuxième partie est estérifiée avec des alcools aliphatiques à longue chaîne linéaire avec des atomes de carbone compris entre 10 et 22, et que les fonctions carboxyles non estérifiées possibles, si présentes, sont salifiées à condition :
quand l'alcool araliphatique est de l'alcool de benzyle et que l'alcool aliphatique se compose d'alcools aliphatiques à longue chaîne linéaire avec des atomes de carbone compris entre C₁₀ et C₂₀, que les fonctions carboxyles estérifiées avec de l'alcool de benzyle soient inférieures à 75 %.

2. Dérivés d'ester selon la revendication 1, **caractérisés par le fait que** l'ester est de l'alcool de benzyle.

3. Dérivés d'ester selon une revendication quelconque parmi les revendications 1 et 2, **caractérisés par le fait que** l'alcool à longue chaîne linéaire est choisi dans le groupe composé d'alcool docosyle, elcosyle, octadécyle, hexadécyle, dodécyle et décyle.

4. Dérivés d'ester selon une revendication quelconque parmi les revendications comprises entre 1 et 3, **caractérisés par le fait que** le pourcentage de fonctions carboxyles d'acide hyaluronique estérifiées avec des alcools araliphatiques varie entre 50 et 75 %.

5. Dérivés d'ester selon une revendication quelconque parmi les revendications comprises entre 1 et 4, **caractérisés par le fait que** le pourcentage de fonctions carboxyles estérifiées avec des alcools aliphatiques à longue chaîne est compris entre 10 et 25 %.

6. Dérivés d'ester selon une revendication quelconque parmi les revendications comprises entre 1 et 6, **caractérisés par le fait que** les fonctions carboxyles restantes sont salifiées avec des métaux alcalins et de terre alcaline et des sels d'ammonium quaternaire.

7. Dérivés d'ester selon la revendication 6, **caractérisés par le fait que** les fonctions carboxyles restantes sont salifiées avec du sodium.

8. Dérivés d'ester selon une revendication quelconque parmi les revendications comprises entre 1 et 7, **caractérisés par le fait que** l'acide hyaluronique de départ a un poids moléculaire compris entre 10.000 et 10.000.000 Da.

9. Dérivés d'ester selon la revendication 8, **caractérisés par le fait que** l'acide hyaluronique a un poids moléculaire compris entre 150.000 et 1.000.000 Da.

10. Fils biodégradables ayant une conformation à plusieurs filaments et une limite d'élasticité comprise entre 300 et 1800 g/cm² composés essentiellement de dérivés d'ester d'acide hyaluronique, **caractérisés par le fait qu'**une première partie de la fonction carboxyle est estérifiée avec un alcool araliphatique, qu'une deuxième partie est estérifiée avec des alcools aliphatiques à longue chaîne linéaire avec des atomes de carbone compris entre 10 et 22 et **caractérisés par le fait que** les fonctions carboxyles non estérifiées possibles, si présentes, sont salifiées.

11. Fils biodégradables selon la revendication 10, **caractérisés par le fait que** l'alcool araliphatique est de l'alcool de benzyle.

12. Fils biodégradables selon une revendication quelconque parmi les revendications 10 et 11, **caractérisés par le fait que** l'alcool aliphatique à longue chaîne linéaire est choisi dans le groupe composé d'alcool docosyle, elcosyle, octadécyle, hexadécyle, dodécyle et décyle.

13. Fils biodégradables selon une revendication quelconque parmi les revendications comprises entre 10 et 12, **caractérisés par le fait que** le pourcentage de fonctions carboxyles d'acide hyaluronique estérifiées avec des alcools araliphatiques varie entre 50 et 75 %.

14. Fils biodégradables selon une revendication quelconque parmi les revendications comprises entre 10 et 13, **caractérisés par le fait que** le pourcentage de fonctions carboxyles estérifiées avec des alcools aliphatiques à longue chaîne est compris entre 10 et 25 %.

15. Fils biodégradables selon une revendication quelconque parmi les revendications comprises entre 10 et 14, **caractérisés par le fait que** les fonctions carboxyles restantes sont salifiées avec des métaux alcalins et de terre alcaline et des sels d'ammonium quaternaire.

16. Fils biodégradables selon la revendication 15, **caractérisés par le fait que** les fonctions carboxyles restantes sont salifiées avec du sodium.

17. Fils biodégradables selon une revendication quelconque parmi les revendications comprises entre 10 et 16, **caractérisés par le fait que** l'acide hyaluronique a un poids moléculaire compris entre 10.000 et 10.000.000 Da.

18. Fils biodégradables selon une revendication quelconque parmi les revendications comprises entre 10 et 16, **caractérisés par le fait que** l'acide hyaluronique a un poids moléculaire compris entre 150.000 et 1.000.000 Da.

19. Fils biodégradables selon une revendication quelconque parmi les revendications comprises entre 10 et 18, **caractérisés par le fait qu'**ils possèdent un diamètre variant entre 75 et 800 microns.

20. Fils biodégradables selon la revendication 19, **caractérisés par le fait qu'**ils ont la forme de fils de suture pour la chirurgie.

21. Matériaux biologiques, produits de soins médicaux et articles de chirurgie sous la forme de compresses de gaze, de filets de mailles, de tissus non tissés, de drains et d'une association de ceux-ci contenant les fils selon une revendication quelconque parmi les revendications comprises entre 10 et 19.

22. Procédé de préparation des fils biodégradables selon une revendication quelconque parmi les revendications comprises entre 10 et 19, comprenant les étapes suivantes :
a) estérification d'une première partie des fonctions carboxyles d'acide hyaluronique avec un alcool araliphatique,
b) estérification des fonctions carboxyles restantes possibles avec des alcools aliphatiques à longue chaîne linéaire avec des atomes de carbone compris entre 10 et 22;
c) salification des fonctions carboxyles restantes possibles d'acide hyaluronique non concernées par les étapes d'estérification précédentes,
d) traitement des esters hyaluroniques mélangés obtenus à l'étape c) selon les procédés de formation des fils conventionnels.

23. Utilisation de dérivés d'ester d'acide hyaluronique, **caractérisée par le fait qu'**une première partie de la fonction carboxyle est estérifiée avec de l'alcool araliphatique, qu'une deuxième partie est estérifiée avec des alcools aliphatiques à longue chaîne linéaire avec des atomes de carbone compris entre 10 et 22, et **caractérisée par le fait que** les fonctions carboxyles non estérifiées possibles, si présentes, sont salifiées pour la fabrication de fils destinés à la chirurgie en général, à la chirurgie maxillo-faciale, à la chirurgie plastique, à la chirurgie esthétique et à la dentisterie.

24. Utilisation de dérivés d'ester d'acide hyaluronique, **caractérisée par le fait qu'**une première partie de la fonction carboxyle est estérifiée avec de l'alcool araliphatique, qu'une deuxième partie est estérifiée avec des alcools aliphatiques à longue chaîne linéaire avec des atomes de carbone compris entre 10 et 20, et **caractérisée par le fait que** les fonctions carboxyles non estérifiées possibles, si présentes, sont salifiées pour la fabrication de fils destinés à être utilisés comme fils de remplissage pour la chirurgie esthétique.
